# EUROPEAN PATENT SPECIFICATION

(11) **EP 2 573 570 B1**
(45) Date of publication and mention of the grant of the patent: **28.03.2018**
(21) Application number: 12184648.9
(22) Date of filing: 17.09.2012
(51) Int. Cl.: G01N 33/74, C08J 5/00, G01N 33/553, G01N 33/58

(54) **Cortisol immunoassay using fluorescent particles**
Cortisolimmunoassay mit fluoreszierenden Partikeln
Dosage immunologique de cortisol utilisant des particules fluorescentes

(30) Priority: 26.09.2011 JP 2011208525; 30.08.2012 JP 2012189776
(43) Date of publication of application: 27.03.2013
(73) Proprietor: Fujifilm Corporation, Minato-ku Tokyo 106-8620 (JP)
(72) Inventor: Kasagi, Noriyuki, Kanagawa, 258-8577 (JP); Watanabe, Yuya, Kanagawa, 258-8577 (JP); Hamasaki, Ryo, Kanagawa, 258-8577 (JP); Nishikawa, Naoyuki, Kanagawa, 258-8577 (JP)
(74) Representative: Hoffmann Eitle

(56) References cited:
- EP-A1- 2 110 658
- GIANFRANCO GIRAUDI ET AL: "Characterisation of cortisol-bovine serum albumin conjugates by chromatofocusing", THE ANALYST, vol. 115, no. 12, 1 January 1990 (1990-01-01), page 1531, XP55049693, ISSN: 0003-2654, DOI: 10.1039/an9901501531
- HOSODA H ET AL: "THE PREPARATION OF STEROID N-HYDROXYSUCCINIMIDE ESTERS AND THEIR REACTIVITIES WITH BOVINE SERUM ALBUMIN", CHEMICAL AND PHARMACEUTICAL BULLETIN,, vol. 27, no. 3, 1 March 1979 (1979-03-01), pages 742-746, XP008056528, ISSN: 0009-2363
- ESKOLA ET AL: "Direct solid-phase time-resolved immunofluorometric assay of cortisol in serum.", CLINICAL CHEMISTRY, vol. 31, no. 10, 1 October 1985 (1985-10-01), pages 1731-1734, XP55050128, ISSN: 0009-9147
- NARA S ET AL: "Colloidal gold probe based rapid immunochromatographic strip assay for cortisol", ANAL. CHIM. ACTA., vol. 682, no. 1-2, 2010, pages 66-71, XP027477332, ISSN: 0003-2670

## Description

### BACKGROUND OF THE INVENTION

### 1. Field of the Invention

The present invention relates to a base plate and a method for immunoassay of cortisol using fluorescent particles.

### 2. Description of the Related Art

Fluorescence detection methods have been hitherto used in a large variety of fields, as analytic methods that are highly sensitive and simple and are capable of quantitatively determining proteins, enzymes, inorganic compounds and the like. These fluorescence detection methods are methods by which a sample which is suspected to contain a substance to be detected (test substance) that is excited by light having a specific wavelength and emits fluorescence, is irradiated with excitation light having the specific wavelength, fluorescence is detected at that time, and thereby the presence of the test substance is verified. Furthermore, in the case where the substance is not a fluorescent material, a method of bringing a substance which specifically binds to a test substance labeled with a fluorescent dye, into contact with a sample, subsequently detecting fluorescence in the same manner as described above, and thereby verifying the presence of this binding, that is, the presence of the test substance, is also widely used.

In regard to such fluorescent detection methods, a method of utilizing the effect of electric field reinforcement by plasmon resonance so as to increase the sensitivity of detection, is known. In such a method, in order to induce plasmon resonance, a sensor chip in which a metal layer is provided in a predetermined region on a transparent support is prepared, and excitation light is caused to enter the sensor chip through the surface of the support on the opposite side of the metal layer-formed surface with respect to the interface between the support and the metal film, at a predetermined angle that is greater than or equal to the total reflection angle. Irradiation of such excitation light causes generation of surface plasmon at the metal layer. Through the electric field reinforcing action brought by the generation of such surface plasmon, fluorescence is reinforced, and thereby the signal/noise ratio (S/N ratio) is increased. As compared with a fluorescence detection method involving surface plasmon excitation (hereinafter, referred to as "SPF method"), in a fluorescence detection method involving epi-illumination excitation (also called epi-illumination fluorescence method), the degree of signal reinforcement can be about 10 times, and analysis can be conducted with high sensitivity.

For example, in the light signal detection method for determining the amount of a test substance as described in JP 2010-190880 A, a sensor chip which has a sensor unit including a metal layer, provided in a predetermined region of one surface of a dielectric plate is prepared, and the sensor unit of the sensor chip is brought into contact with a sample. Through such contact, a binding substance attached with a photoresponsive labeling substance in an amount equivalent to the amount of the test substance contained in the sample, binds to the sensor unit. Subsequently, light from the photoresponsive labeling substance, which is produced in the electric field-reinforced field produced on the metal layer by irradiating excitation light to the predetermined region, is detected, and thereby the amount of the test substance can be determined. Furthermore, in this method, it is also possible to use, as a photoresponsive labeling substance, a light transmitting material which transmits the light produced from a photoresponsive substance, and in which plural photoresponsive substances are included such that the metal quenching occurring when the photoresponsive substances approach the metal layer is prevented.

Cortisol, which is one kind of adrenocortical hormones, is a kind of glucocorticoid that controls the metabolism of carbohydrates, fats and proteins. Among three kinds of glucocorticoids, cortisol is available in the largest amount in the body. It is known that an increase in the secretion of cortisol is also caused by stress, and depending on the amount of secretion, there may be effects such as a increase in the blood pressure or the blood glucose level, or a decrease in the immune function. Furthermore, cortisol (also called hydrocortisone) is clinically used as a steroidal anti-inflammatory drug, and may be used in the treatment of, for example, acute inflammation, chronic inflammation, autoimmune diseases, allergic diseases, shock, gout, acute leukemia, and allograft rejection. As an immunoassay method for cortisol, for example, an analysis method based on immunochromatoassay is known (Anal. Chim. Acta., 2010, 682(1-2), 66-71).
Giraudi and Baggiani have reported the characterisation of cortisol bovine serum albumin conjugates by chromatofocusing (Analyst, December 1990, volume 115, page 1531). Ishii et al. have reported the preparation of steroid N-hydroxysuccinimide esters and their reactivity with bovine serum albumin (Chem. Pharm. Bull., 1979, 27, 742-747). Eskola et al. have reported a direct solid-phase time-resolved immunofluorometric assay of cortisol in serum (Clin. Chem., 1985, 31/10, 1731-1734). EP-A-2 110 658 discloses an optical signal detection method for detecting a specific substance in a sample by detecting an optical signal output from a label. Nara et al. have reported a colloidal gold probe based rapid immunochromatographic strip assay for cortisol (Analytica Chimica Acta, 2010, 68, 66-71).

### SUMMARY OF THE INVENTION

As discussed above, for example, an analysis method based on immunochromatoassay is known as an immunoassay method for cortisol; however, development of an immunoassay method which exhibits higher sensitivity is desired. An object to be solved by the present invention is to provide a base plate and a method for immunoassay of cortisol, which enable immunoassay of cortisol with high sensitivity in a cortisol concentration range of particular clinical significance (that is, 2 µg/dL to 30 µg/dL).

The inventors of the present invention conducted a thorough investigation to solve the problems described above, and as a result, they found that cortisol can be analyzed by immunoassay with high sensitivity in a clinically significant cortisol concentration range (that is, 2 µg/dL to 30 µg/dL), by using a base plate on which a cortisol albumin conjugate obtained by conjugating cortisol and albumin at certain proportions is immobilized, bringing this base plate into contact with anti-cortisol antibody-labeled fluorescent particles and cortisol as a test substance, so as to cause the cortisol on the base plate (cortisol in the cortisol/albumin conjugate) and the cortisol as a test substance to competitively bind to the anti-cortisol antibody-labeled fluorescent particles, and thereby measuring the fluorescence based on the anti-cortisol antibody-labeled fluorescent particles bound to the base plate. The present invention was accomplished based on these findings.

Specifically, according to an aspect of the present invention, there is provided a base plate for cortisol immunoassay on which a cortisol albumin conjugate having a cortisol/albumin ratio of from 12 to 20 is immobilized.

According to another aspect of the present invention, there is provided a method for measuring cortisol in a test sample, the method including:
(1) a step of bringing anti-cortisol antibody-labeled fluorescent particles and a test sample containing cortisol into contact with
   a base plate for cortisol immunoassay on which a cortisol albumin conjugate having a cortisol/albumin ratio of from 12 to 20 is immobilized; and
(2) a step of measuring the fluorescence based on the anti-cortisol antibody-labeled fluorescent particles bound to the base plate.

Preferably, the albumin is a bovine serum albumin.

Preferably, the cortisol albumin conjugate is a cortisol albumin conjugate having a cortisol/albumin ratio of from 14 to 18.

Preferably, the cortisol albumin conjugate is immobilized on a metal film provided on the base plate.

Preferably, in the step (2), fluorescence is measured by surface plasmon fluorescence measurement or epi-illumination fluorescence measurement.

According to the present invention, cortisol can be analyzed by immunoassay with high sensitivity in a clinically significant cortisol concentration range (that is, 2 µg/dL to 30 µg/dL).

### BRIEF DESCRIPTION OF THE DRAWINGS

Fig. 1 is a diagram illustrating the relationship between the cortisol concentration and the normalized value of fluorescence signal, B/B0, in the fluorescence measurement using Conjugate 1 (cortisol/albumin ratio is 15.7) (Example of the present invention).
Fig. 2 is a diagram illustrating the relationship between the cortisol concentration and the normalized value of fluorescence signal, B/B0, in the fluorescence measurement using Conjugate 2 (cortisol/albumin ratio is 11.0) (Comparative Example 1).
Fig. 3 is a diagram illustrating the relationship between the cortisol concentration and the normalized value of fluorescence signal, B/B0, in the fluorescence measurement using Conjugate 3 (cortisol/albumin ratio is 7.0) (Comparative Example 2).

### DESCRIPTION OF THE PREFERRED EMBODIMENTS

Hereinafter, the present invention will be described in more detail.

The present invention relates to a a base plate for cortisol immunoassay on which a cortisol albumin conjugate having a cortisol/albumin ratio of from 12 to 20 is immobilized, and a method for measuring cortisol in a test sample, the method including (1) a step of bringing anti-cortisol antibody-labeled fluorescent particles and a test sample containing cortisol into contact with the base plate; and (2) a step of measuring the fluorescence based on the anti-cortisol antibody-labeled fluorescent particles bound to the base plate.

### (Cortisol)

Cortisol is a glucocorticoid having the following structure:

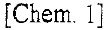

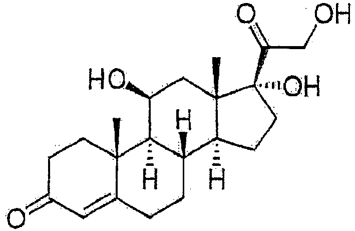

The type of albumin used in the present invention is not particularly limited, but for example, albumin derived from an animal (cattle, human being or the like), preferably a serum albumin derived from an animal (cattle, human being or the like), and particularly preferably bovine serum albumin (BSA) or the like can be used.

### (Cortisol-albumin conjugate)

The cortisol/albumin ratio in the conjugate used in the present invention is from 12 to 20, preferably from 14 to 18, and most preferably from 15 to 17. When the cortisol/albumin ratio is greater than 20, solubility in water or organic solvents is decreased, and the decreased solubility affects the accuracy of measurement. Furthermore, when the cortisol/albumin ratio is less than 12, the immunoassay performance becomes poor, and the effect of the present invention cannot be achieved.

The cortisol/albumin ratio means a ratio of the number of molecules (mole number), and means the number of cortisol molecules bound to one molecule of albumin (BSA or the like). The cortisol/albumin ratio can be determined by, for example, matrix-assisted laser desorption-ionization time-of-flight mass spectrometry (MALDI-TOF-MS), and the specific procedure of measurement is as follows. Here, the procedure of measurement in the case of using BSA as the albumin will be described. A sample is dissolved in 0.1 mass% trifluoroacetic acid (TFA):acetonitrile (ACN) = 2/1, and the concentration is adjusted to 1 mg/mL. 4 µL of a matrix (sinapinic acid (SA)) and 1 µL of the sample are mixed, and the mixture is spot-applied in 1 µL × 4 spots on a gold plate. Thereafter, the sample is naturally dried. The gold plate is inserted into a MALDI-TOF-MS apparatus (Voyger manufactured by Applied Bio Systems, Inc.), and measurement is carried out Accumulated data of 900 shots are obtained from each spot (N = 4). In regard to a peak corresponding to the cortisol-BSA conjugate, the position vertically drawn from the center of the area of the part which exhibits an intensity of 50% or greater of the maximum value of the peak intensity of the peak is designated as the molecular weight of the cortisol-BSA conjugate, and the average value of N = 4 is used to calculate the number of bound molecules according to the formula: (molecular weight of cortisol-BSA conjugate - molecular weight of naive BSA)/molecular weight of the cortisol derivative (435 - 18 = 417).

Meanwhile, an example of commercially available cortisol albumin conjugates is Catalogue No.: H-2384 of Sigma-Aldrich Co., Hydrocortisone 3-(O-carboxymethyl)oxime:BSA (26 mol steroid/mol BSA). However, the cortisol albumin ratio of this product is 26, and this product has low solubility in water or organic solvents, and could not be applied to the measurement of the present invention.

The cortisol albumin conjugate used in the present invention may be a conjugate in which cortisol and albumin are directly bonded to each other, or may be a conjugate in which cortisol and albumin are bonded via an appropriate linker. For example, as will be described in the following Examples, a cortisol albumin conjugate can be produced by producing an oxime derivative of cortisol having a carboxyl group, activating this carboxyl group by using 1-ethyl-3-(3-dimethylaminopropyl)carbodiimide (EDC), N-hydroxysuccinimide (NHS) and the like, and bonding the activated carboxyl group to an amino group of the albumin.

The cortisol/albumin ratio for the conjugate used in the present invention can be regulated by adjusting the reaction conditions for the bonding reaction between cortisol and albumin (the use amounts of cortisol and albumin, and the like). The aforementioned adjustment can be carried out by making reference to the literature (Bioconjugate Chem., 1994, 4, 419-424).

### (Base plate)

In the present invention, a base plate for cortisol immunoassay is provided by immobilizing the above-described cortisol albumin conjugate having a cortisol/albumin ratio of from 12 to 20, to a base plate. The type of the base plate is not particularly limited so long as the base plate is capable of the fluorescence analysis that will be described below, and any blank base plate can be used. The cortisol albumin conjugate can be bonded to the base plate by a method of dissolving the conjugate in a buffer solution, spot-applying the solution onto the base plate, leaving the solution to stand for a certain time, subsequently suctioning the supernatant, and drying the residue.

In the case of performing surface plasmon fluorescence (SPF) detection that will be described below, regarding the base plate, it is preferable to use a base plate having a metal film on the surface. The metal that forms the metal film is not particularly limited so long as the metal is capable of producing surface plasmon resonance. Preferred examples thereof include free-electron metals such as gold, silver, copper, aluminum, and platinum, and gold is particularly preferred. Those metals can be used individually or in combinations. Furthermore, in consideration of the adhesiveness to the base plate, an intermediate layer formed from chromium or the like may be provided between the base plate and the layer formed of a metal. The thickness of the metal layer is not limited, but for example, the thickness is preferably from 0.1 nm to 500 nm, and particularly preferably from 1 nm to 200 nm. When the thickness is greater than 500 nm, the surface plasmon phenomenon of the medium cannot be sufficiently detected. Also, in the case of providing an intermediate layer formed from chromium or the like, the thickness of the intermediate layer is preferably from 0.1 nm to 10 nm.

Formation of the metal film may be carried out according to a routine method, and can be carried out by, for example, a sputtering method, a vapor deposition method, an ion plating method, an electroplating method, or an electroless plating method.

The metal film is preferably disposed on the base plate. Here, the term "disposed on the base plate" includes the case where the metal film is disposed on the base plate so as to be in direct contact, as well as the case where the metal film is disposed over the base plate without being in direct contact with the base plate, with another layer interposed between the metal film and the base plate. Regarding the material of the base material that can be used in the present invention, for example, in the case of using the base plate for a surface plasmon resonance biosensor, a material which is transparent to laser light, such as optical glass such as BK7 (borosilicate glass), which is one kind of general optical glass, or a synthetic resin, specifically, polymethyl methacrylate, polyethylene terephthalate, polycarbonate or a cycloolefin polymer, can be used. For such a base plate, preferably, a material which does not exhibit anisotropy to polarized light and has excellent processability is desirable.

An example of the base plate for SPF detection may be a base plate obtained by vapor depositing a gold film on polymethyl methacrylate (PMMA).

### (Fluorescent particles)

Furthermore, according to the present invention, cortisol in a test sample can be measured by bringing the base plate for cortisol immunoassay of the present invention as described above, into contact with anti-cortisol antibody-labeled fluorescent particles and a test sample containing cortisol, and then measuring the fluorescence originating from the anti-cortisol antibody-labeled fluorescent particles bound to the cortisol albumin conjugate on the base plate.

Particles colored with a fluorescence that can be conventionally used in an immune reaction can be used as the fluorescent particles to be used in the present invention, and for example, fluorescent polymer particles such as fluorescent polystyrene beads, and fluorescent glass particles such as fluorescent glass beads can be used. Specific examples of the material of the fluorescent particles include synthetic polymer powders of polymers using monomers such as styrene, methacrylic acid, glycidyl (meth)acrylate, butadiene, vinyl chloride, vinyl acetate acrylate, methyl methacrylate, ethyl methacrylate, phenyl methacrylate, and butyl methacrylate; and copolymers using two or more monomers. Latexes obtained by uniformly suspending these synthetic polymer powders are preferred. Further examples include organic polymer powders, inorganic substance powders, microorganisms, blood corpuscles, cellular membrane fragments, and liposomes.

In the case of using latex particles, specific examples of the latex material include polystyrene, a styrene-acrylic acid copolymer, a styrene-methacrylic acid copolymer, a styrene-glycidyl (meth)acrylate copolymer, a styrene-styrene sulfonate copolymer, a methacrylic acid polymer, an acrylic acid polymer, an acrylonitrile-butadiene-styrene copolymer, a vinyl chloride-acrylic acid ester copolymer, and polyvinyl acetate acrylate. The latex is preferably a copolymer containing at least styrene as a monomer, and particularly preferably a copolymer of styrene with acrylic acid or methacrylic acid. The method for producing the latex is not particularly limited, and the latex can be produced by any polymerization method. However, if a surfactant is present at the time of antibody labeling, antibody immobilization is not easily achieved. Therefore, for the production of the latex, emulsifier-free emulsion polymerization, that is, emulsion polymerization in which no emulsifier such as surfactant is used, is preferred.

When the latex which is obtained by polymerization is fluorescent per se, the latex can be used directly as fluorescent latex particles. When the latex obtained by polymerization is non-fluorescent, fluorescent latex particles can be produced by adding a fluorescent substance (a fluorescent dye or the like) to the latex. That is, fluorescent latex particles can be produced by adding a fluorescent dye to a solution of latex particles containing water and a water-soluble organic solvent, and stirring the mixture.

The average particle size of the fluorescent particles may vary with the material of the particles, the concentration range for the quantitative determination of the test substance, the measuring instrument, and the like; however, the average particle size is preferably in the range of 0.001 µm to 10 µm (more preferably 0.001 µm to 1 µm). Liposomes or microcapsules containing a fluorescent dye can also be used as fluorescent particles. The fluorescence emission color is not particularly limited so long as the color is to be emitted when the fluorescent substance is excited by absorbing ultraviolet radiation or the like, and returns to the ground state, and for example, fluorescence emission colors such as yellow-green (excitation wavelength: 505 nm/emission wavelength: 515 nm; hereinafter, the same), blue (350 nm to 356 nm/415 nm to 440 nm), red (535 nm to 580 nm/575 nm 605 nm), orange (540 nm/560 nm), red-orange (565 nm/580 nm), crimson (625 nm/645 nm), and dark red (660 nm/680 nm) can be used. Fluorescent particles emitting these fluorescence spectra are available from, for example, Invitrogen, Inc., and those fluorescent particles are commercially available under the trade name FluoSpheres (registered trademark) from the same company.

### (Method for measuring average particle size)

The average particle size of the fluorescent particles used in the present invention can be measured with a commercially available particle size distribution analyzer or the like. Known examples of the measurement methods for particle size distribution include optical microscopy, confocal laser microscopy, electron microscopy, atomic force microscopy, static light scattering, laser diffraction, dynamic light scattering, centrifugal sedimentation, electric pulse measurement, chromatography and ultrasonic attenuation, and apparatuses corresponding to the various principles are commercially available.

In view of the particle size range and the ease of measurement, a dynamic light scattering method can be preferably used in the present invention. Examples of commercially available measuring apparatuses using dynamic light scattering include NanoTrack UPA (Nikkiso Co., Ltd.), a dynamic light scattering type particle size distribution analyzer, LB-550 (Horiba, Ltd.), and a concentrated system particle size analyzer, FPAR-1000 (Otsuka Electronics Co., Ltd.). In the present invention, the average particle size is determined as the value of a median diameter (d = 50) measured at a measurement temperature of 25°C.

### (Anti-cortisol antibody)

As the anti-cortisol antibody to be bonded to the fluorescent particles, an antibody exhibiting specificity to cortisol may be used. Examples of the anti-cortisol antibody that can be used include an antiserum prepared from the blood serum of an animal that has been immunized with cortisol, an immunoglobulin fraction purified from an antiserum, a monoclonal antibody obtainable by cell fusion using the spleen cells of an animal that has been immunized with cortisol, and fragments thereof [for example, F(ab')2, Fab, Fab', and Fv]. Preparation of these antibodies can be carried out by conventional methods. Furthermore, the antibody may be modified as in the case of a chimeric antibody or the like, and a commercially available antibody or an antibody prepared from an animal blood serum or a culture supernatant by a known method can also be used.

The antibody can be used without being restricted by the animal species or the subclass. For example, an antibody that can be used in the present invention is an antibody derived from an organism which can have an immune reaction, such as mouse, rat, hamster, goat, rabbit, sheep, cattle, or chicken, and specific examples thereof include mouse IgG, mouse IgM, rat IgG, rat IgM, hamster IgG, hamster IgM, rabbit IgG, rabbit IgM, goat IgG, goat IgM, sheep IgG, sheep IgM, bovine IgG, bovine IgM, and bird IgY. These antibodies are applicable to both polyclonal and monoclonal antibodies. A fragmentized antibody is a molecule derived from a whole antibody, having at least one antigen binding site, and specific examples thereof include Fab and F(ab')2. These fragmentized antibodies are molecules obtainable by using an enzymatic or chemical treatment, or a genetic engineering technique.

Methods for immobilizing a binding substance such as an antibody or an antigen to particles are described in, for example, JP 2000-206115 A or the protocol attached to FluoSpheres (registered trademark) Polystyrene Microsphere F8813 of Molecular Probe, Inc., and any known methods for preparing a reagent for immune agglutination can all be used. Furthermore, any principle for physical adsorption or chemical bonding by covalent bonding can be employed as the principle for immobilizing an antibody as a binding substance to particles. As a blocking agent which is used to cover the surfaces of particles that are not coated with an antibody after the antibody is immobilized to particles, any known substances, for example, bovine serum albumin (BSA), skimmed milk, casein, a soybean-derived component, a fish-derived component, polyethylene glycol, and commercially available blocking agents for immune reaction containing these substances or equivalent substances, can be used. These blocking agents can be subjected to a pretreatment such as partial modification by heat, an acid or an alkali as necessary.

A specific method for immobilizing an antibody to particles will be illustrated below. An antibody solution having the concentration adjusted to 0.01 mg/mL to 20 mg/mL is added to a liquid prepared by dispersing particles to a solids concentration of 0.1% to 10%, and the mixture is mixed. Stirring is continued for 5 minutes to 48 hours under the temperature conditions of 4°C to 50°C. Subsequently, the particles are separated from the solution by centrifugation or any other method, and any antibody that is contained in the solution and has not been bound to the particles is sufficiently removed. Thereafter, an operation of washing the particles with a buffer solution is repeated 0 to 10 times. After the operation of mixing the particles and the antibody and causing the antibody to bind to the particles is carried out, it is desirable to protect the part on the particle surface that is not bound by the antibody, by using any component which does not participate in the antigen-antibody reaction, preferably a protein, and more preferably a blocking agent such as BSA (bovine serum albumin), Block-Ace, skimmed milk or casein.

When an antigen or an antibody is immobilized to particles, a stabilizer can be added as necessary. The stabilizer is not particularly limited so long as it is a compound which stabilizes an antigen or an antibody, such as sucrose or a synthetic or natural polymer such as a polysaccharide, and a commercially available product such as Immunoassay Stabilizer (Applied Biosystems, Inc.) can also be used.

### (Measurement method)

The measurement method of the present invention is construed by the broadest concept, including the detection of possible presence of cortisol and the measurement of the amount of cortisol (that is, quantification). A specific embodiment of the measurement method of the present invention may be a competition method.

In the competition method according to the present invention, first, a base plate for cortisol immunoassay on which a cortisol albumin conjugate having a cortisol/albumin ratio of from 12 to 20 is immobilized, is brought into contact with anti-cortisol antibody-labeled fluorescent particles and a test sample containing cortisol. When cortisol is not present in the test sample, an antigen-antibody reaction occurs on the base plate between the anti-cortisol antibody-labeled fluorescent particles and the cortisol on the base plate (that is, cortisol in the cortisol albumin conjugate). On the other hand, when cortisol is present in the test sample, an antigen-antibody reaction occurs between the cortisol in the test sample and the anti-cortisol antibody-labeled fluorescent particles, and the antigen-antibody reaction between the anti-cortisol antibody-labeled fluorescent particles and the cortisol on the base plate (that is, cortisol in the cortisol albumin conjugate) is inhibited. After the reaction described above is completed, the anti-cortisol antibody-labeled fluorescent particles that are not bound to the albumin on the base plate are removed. Subsequently, the extent of formation of an immune complex (that is, a complex between the anti-cortisol antibody-labeled fluorescent particles and the cortisol in the cortisol albumin conjugate on the base plate) on the base plate is detected, and thereby the concentration of cortisol in the test sample can be measured.

In the competition method, the form of fluorescence measurement may be plate reader measurement or may be flow measurement, but the measurement can be carried out by, for example, the following method. Plural samples with known amounts of cortisol and with different cortisol concentrations are prepared in advance, and each of these samples and anti-cortisol antibody-labeled fluorescent particles are mixed in advance. This liquid mixture is brought into contact with a region where a cortisol albumin conjugate is immobilized. The fluorescence signal from the region where the cortisol albumin conjugated is measured as plural fluorescence signals while the liquid mixture is brought into contact with the conjugate at a specific time interval. From these plural fluorescence signals, the change in the amount of fluorescence over time (gradient) is determined for various cortisol concentrations. This time change is plotted on the Y-axis, while the cortisol concentration is plotted on the X-axis, and a relationship formula for the cortisol concentration against the change in the amount of fluorescence over time is obtained by using an appropriate fitting method such as a least-squares method. Based on the relationship formula obtained as described above, the amount of cortisol contained in the test sample can be quantitatively determined by the results of the change in the amount of fluorescence over time obtained by using a test sample intended for examination.

### (Measurement of surface plasmon fluorescence)

The method for detecting fluorescence in the present invention is not particularly limited, but it is preferable to detect fluorescence intensity by using, for example, an instrument capable of detecting fluorescence intensity, specifically, a microplate reader or a biosensor for carrying out the detection of fluorescence caused by surface plasmon excitation (SPF). The detection method for fluorescence caused by surface plasmon excitation (SPF method) can make measurement with higher sensitivity than a detection method for fluorescence caused by epi-illumination excitation (hereinafter, referred to as "epi-illumination fluorescence method").

Regarding the surface plasmon fluorescence (SPF) biosensor, for example, a sensor described in JP 2008-249361 A, which includes a optical waveguide formed of a material which transmits excitation light having a predetermined wavelength; a metal film formed on one surface of this optical waveguide; a light source that generates a light beam; an optical system which causes the light beam to enter through the optical waveguide at an incident angle at which surface plasmon is generated with respect to the interface between the optical waveguide and the metal film; and a fluorescence detection means that detects the fluorescence generated as a result of excitation caused by the evanescent wave reinforced by the surface plasmon, can be used.

The detection system for fluorescence caused by surface plasmon excitation (SPF) using the fluorescent particles of the present invention is an assay method for detecting fluorescence from a fluorescent substance depending on the amount of a test substance immobilized on a metal thin film on a base plate, and is a method different from a so-called latex agglutination method which detects the change in the optical transparency resulting from the progress of the reaction in a solution, for example, as turbidity. In the latex agglutination method, an antibody-sensitized latex in a latex reagent and the antigen in a specimen bind to each other through an antibody reaction, and agglutinate. This agglutinate increases with time, and a method of quantitatively determining the antigen concentration from the change in absorbance per unit time obtainable by irradiating near-infrared radiation to this agglutinate, constitutes the latex agglutination method. In the present invention, a detection method for a test substance, which is very simple compared to the latex agglutination method, is provided.

The present invention will be more specifically described by way of the following Examples, but the present invention is not intended to be limited by the Examples.

### Examples

### (Example 1)

### 1. Preparation of cortisol-BSA conjugate

### 1-1. Synthesis of cortisol oxime derivative 1

According to the literature (Steroids, 1974, Jan. 49-64), synthesis of a cortisol oxime derivative 1 was carried out by enaminating cortisol (may be abbreviated to COR or Corti), causing the product to react with a hydroxylamine derivative. The reaction scheme is shown below.

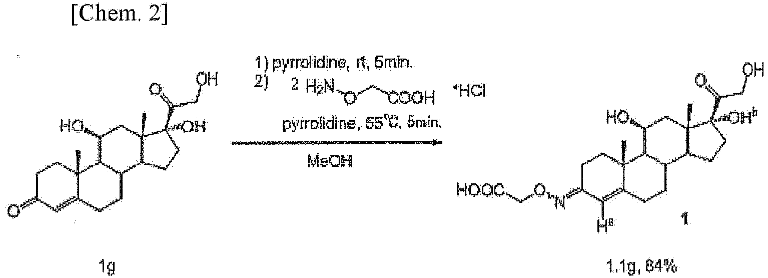

One equivalent of pyrrolidine (manufactured by Wako Pure Chemical Industries, Ltd.) was added dropwise to a methanol suspension of cortisol (1 g, manufactured by Wako Pure Chemical Industries, Ltd.), and the mixture was stirred to 5 minutes at room temperature. Enamine was produced in the reaction system, and the solution turned red. Thereafter, when another 1 equivalent of pyrrolidine and 1 equivalent of a hydroxylamine derivative (manufactured by Wako Pure Chemical Industries, Ltd.) were added thereto, the color disappeared. The reaction temperature was adjusted to 55°C, and the system was stirred to implement oximation. The reaction proceeded smoothly, and a cortisol oxime derivative 1 (1.1 g; 84%) was obtained.

### 1-2. Preparation of cortisol-BSA conjugate

A cortisol-BSA conjugate was produced by the following method, by using the cortisol oxime derivative 1 synthesized in section 1-1. The reaction scheme is shown below.

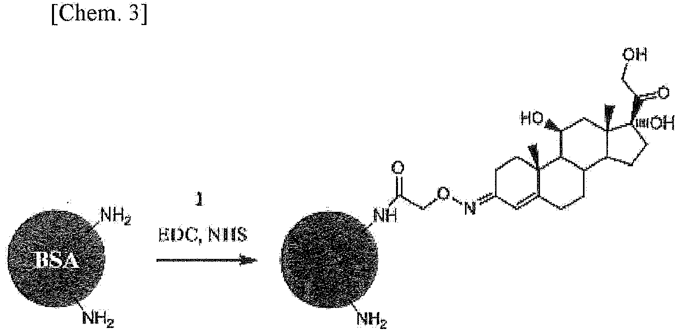

The cortisol oxime derivative 1 (50 mg, 115 µm) in dry dimethylformamide (DMF, manufactured by Wako Pure Chemical Industries, Ltd.) was subjected to active esterification by using excess amounts of NHS (manufactured by Wako Pure Chemical Industries, Ltd.) and EDC (manufactured by Wako Pure Chemical Industries, Ltd.). Thereafter, the resultant was added dropwise to a phosphate buffer solution (PBS, manufactured by Wako Pure Chemical Industries, Ltd.) of BSA (bovine serum albumin, manufactured by Wako Pure Chemical Industries, Ltd., 193 mg), which is one kind of albumin. After completion of the reaction, the reaction solution was purified by dialysis in an ACN (manufactured by Wako Pure Chemical Industries, Ltd.)/water = 1/1 (containing 0.1 mass% TFA (manufactured by Wako Pure Chemical Industries, Ltd.)) solution (5 L). Finally, the solution was lyophilized, and thus a white solid was obtained (this was designated as conjugate 1). Conjugate 2 and conjugate 3 were obtained in the same manner, except that the use amount of BSA and the amount of PBS were changed as indicated in Table 1.

### 1-3. Measurement of cortisol/BSA ratio (cortisol/albumin ratio) by MALDI-TOF-MS

### (Measurement procedure)

The cortisol-BSA conjugate prepared in section 1-2. was dissolved in a solution of 0.1 mass% TFA:ACN = 2/1, and the solution was adjusted to a concentration of 1 mg/mL. 1 µL of this solution and 4 µL of a matrix (SA; sinapinic acid) were mixed, and the mixture was spot-applied in 1 µL × 4 points on a gold plate. The mixture was naturally dried, and then the gold plate was inserted into a MALDI-TOF-MS apparatus (Voyger manufactured by Applied Bio Systems, Inc.). Accumulated data of 900 shots are obtained from each spot (N = 4) to obtain the mass information. By using these data, the center value of the molecular weight at 50% intensity of the maximum value of the peak intensity of a peak corresponding to the cortisol-BSA conjugate was employed as the peak of the BSA conjugate, and the position vertically drawn from this peak value was designated as the molecular weight of the cortisol-BSA conjugate. The average value of N = 4 was used to calculate the binding number of cortisol to BSA by the formula: (molecular weight of cortisol-BSA conjugate - molecular weight of naive BSA)/molecular weight of the cortisol derivative (435 - 18 = 417). An example of the intensity of the MS spectrum is shown below, and the cortisol/BSA ratios of the conjugates 1, 2 and 3 thus obtained are indicated in Table 1.

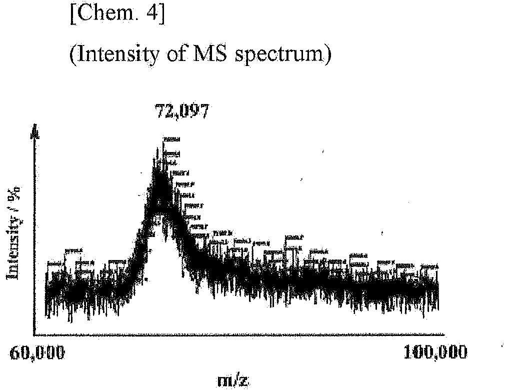

### [Table 1]

**Table 1: Summary of preparation of cortisol (Corti)-BSA conjugates**

| | Conjugate 1 | Conjugate 2 | Conjugate 3 |
|---|---|---|---|
| BSA | 193 mg | 322 mg | 643 mg |
| Corti derivative | 50 mg | 50 mg | 50 mg |
| PBS | 39 mL | 65 mL | 130 mL |
| Corti/BSA* | 15.7 | 11.0 | 7.0 |

| | | | |
|---|---|---|---|
| * The Corti/BSA ratio was determined by MALDI-TOF-MS described below. | | | |

### 2. Preparation of fluorescent particles labeled with anti-cortisol antibody

Fluorescent particles labeled with an anti-cortisol antibody were prepared in the following manner.

250 µL of a 50 mM MES buffer (pH 6.0) solution was added to 250 µL of a 2 mass% (solids concentration) aqueous solution of fluorescent latex particles (manufactured by Invitrogen, Inc.; average particle size 200 nm), and 100 µL of 5 mg/mL an anti-cortisol monoclonal antibody (manufactured by Hytest, Ltd.; Anti-Cortisol MAb XM210) was added thereto. The mixture was stirred for 15 minutes at room temperature. Thereafter, 5 µL of a 10 mg/mL aqueous EDC solution was added thereto, and the mixture was stirred for 2 hours at room temperature. 25 µL of a 2 mol/L aqueous solution of glycine (manufactured by Wako Pure Chemical Industries, Ltd.) was added thereto, and the mixture was stirred for 30 minutes. Subsequently, the mixture was centrifuged (15,000 rpm, 4°C, 15 minutes), and thus fluorescent latex particles were allowed to sediment. Thereafter, the supernatant was removed, 500 µL of a PBS solution (pH 7.4) was added to the particles, and the fluorescent latex particles were redispersed by using an ultrasonic cleaning machine. Again, the dispersion was centrifuged (15,000 rpm, 4°C, 15 minutes), and the supernatant was removed. Subsequently, the fluorescent latex particles were redispersed by adding 500 µL of a PBS (pH 7.4) solution containing 1 mass% BSA was added to the particles. Thus, a 1 mass% solution of anti-cortisol antibody-bound fluorescent latex particles was prepared.

### 3. Production of flow channel type sensor chip

### 3-1. Production of cortisol-BSA conjugate-immobilized base plate

A base of polymethyl methacrylate (PMMA) (manufactured by Mitsubishi Rayon Co., Ltd., Acrypet VH) was provided, and a gold film having a thickness of 50 nm was deposited on one surface of the base by a vapor deposition method to a width of 4 mm. Thus, a chip for constructing a base plate was produced. On the gold-deposited surface of this chip, a liquid containing the conjugate 1 (Example of the present invention), conjugate 2 (Comparative Example 1) or conjugate 3 (Comparative Example 2) (concentration: 50 µg/mL in 50 mM MES buffer solution, pH 6, 150 mM NaCl) was spot-applied and dried. Thus, a plural number of base plates 1 to 3, on which each of the conjugates was immobilized, were produced.

### 3-2. Washing of base plate

Each of the three types of base plates produced as such were repeatedly washed for three times by using 300 µL of a washing solution prepared in advance (a PBS solution (pH 7.4) containing 0.05 mass% Tween 20 (polyoxyethylene(20) sorbitan monolaurate, manufactured by Wako Pure Chemical Industries, Ltd.)).

### 3-3. Production of flow channel type sensor chip

Flow channel type sensor chips were produced by encapsulating the three kinds of base plates produced according to the configuration of the second exemplary embodiment of JP 2010-190880 A, in flow channels.

### 4. Immunoassay of cortisol using fluorescent particles

Samples containing cortisol at various concentrations (1.1 µg/dL, 2.0 µg/dL, 3.2 µg/dL, 9.0 µg/dL, 24.6 µg/dL, and 29.0 µg/dL) were prepared, and each of the samples was mixed in advance with the anti-cortisol antibody-labeled fluorescent particles prepared in section 2. while the mixture was stirred for 10 minutes. Subsequently, the mixture was spot-applied on the flow channel type sensor chips encapsulating the base plates 1 to 3 produced in section 3-3. After the spot-application, the mixture liquid was caused to flow down at a rate of 10 µL/min while pump suction was performed, and the fluorescence intensity of the gold-deposited surface where the cortisol-BSA conjugate was immobilized was continuously measured for 1.5 minutes. For each of the base plates, the rate of increase in the fluorescence intensity thus obtained per unit time was determined as the fluorescence signal value.

A sample having a cortisol concentration of zero was prepared in the same manner, and the fluorescence signal value determined with the sample was designated as B0, while the signal value of each of the samples containing cortisol was designated as B. The signal value was normalized by determining the ratio B/B0.

### 5. Calibration curve

It is described in the literature "The Immunoassay Handbook, Third Edition, Edited by David Wild (2005)" that a four-parameter logistic curve model of a sigmoid function can be applied as the calibration curve for a competition method. Thus, according to this method, a four-parameter logistic curve passing the nearest neighbor points of the respective points of the fluorescence signal values obtained at the various cortisol concentrations measured in section 4. was determined by using the least squares method that is generally known as a method for obtaining an approximate curve. This curve was used as the calibration curve.

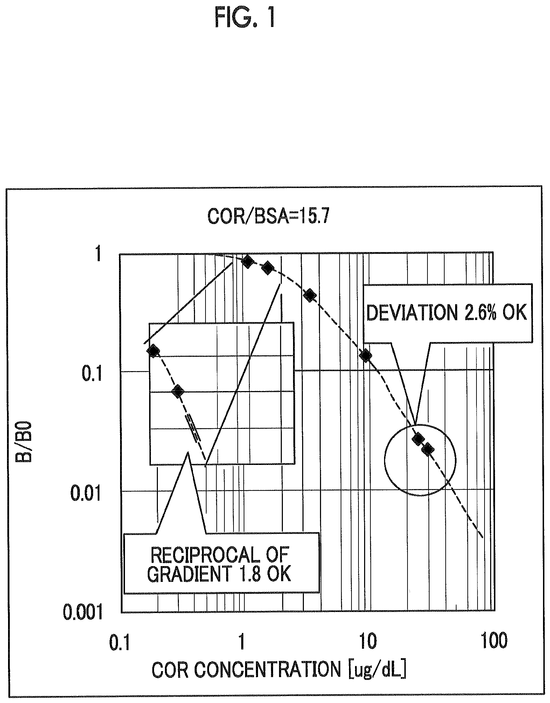

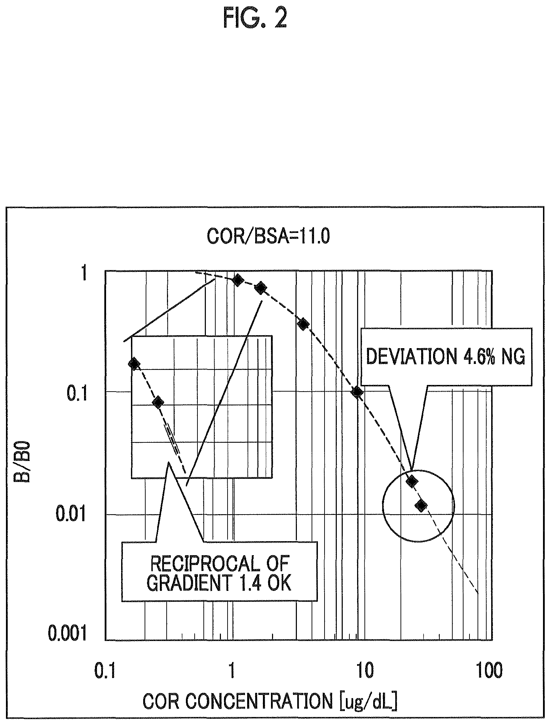

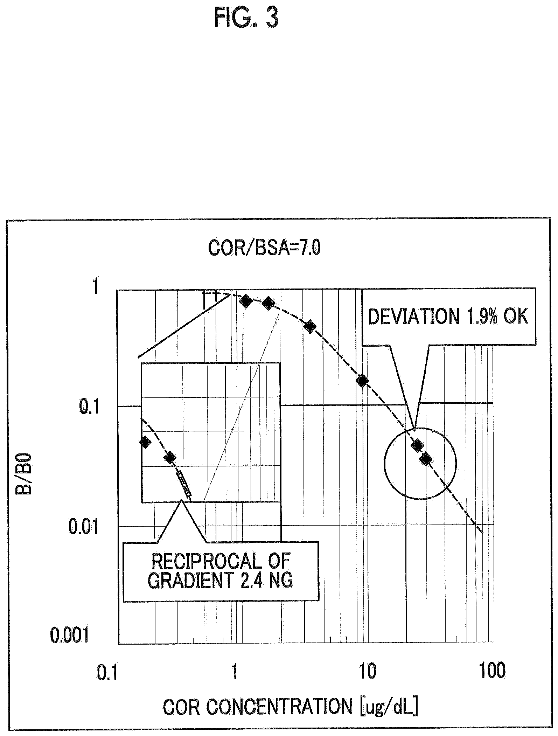

The calibration curve determined as described above and the average values of the measured values of the respective cortisol concentrations are shown in Fig. 1 to Fig. 3 for each of the cortisol/BSA ratio (indicated as COR/BSAin the diagrams).

The performance of the cortisol-BSA conjugate was determined based on whether the standard of the calibration curve was satisfied. The calibration curve was defined by two items. The first item was the gradient of the calibration curve in a low concentration range, and when the reciprocal number of the gradient was calculated, a curve exhibiting a reciprocal number of 2.0 or less was considered as a standard. The second item was the deviation from the calibration curve at a measurement point in a high concentration range, and a curve exhibiting a deviation of 4% or less was considered as a standard. Within this range, the coefficient of fluctuation of the measured values was 10% or less, and an accuracy of 10% or less could be achieved. Thus, highly accurate measurement is made possible.

The gradient of the calibration curve at 2.0 µg/dL, which was the minimum concentration of cortisol that was clinically significant, was determined for the low concentration range. Furthermore, for the high concentration range, the deviations from the respective calibration curves at cortisol concentrations of 24.6 µg/dL and 29.0 µg/dL were determined, and the average value was calculated to perform an evaluation. The results are summarized in Table 2.

### [Table 2]

**Table 2: Gentleness of calibration curve in low concentration range, and deviation of average measured values from calibration curves in high concentration range**

| Cortisol/BSA ratio | Reciprocal of calibration curve gradient in low concentration range | | Deviation from calibration curve in high concentration range | | |
|---|---|---|---|---|---|
| | (Reference 2.0 or less | Result | (Reference 4% or less) | Result | |
| 15.7 | 1.8 | Good | 2.6% | Good | Example |
| 11.0 | 1.4 | Good | 4.6% | NG | Comparative Example |
| 7.0 | 2.4 | NG | 1.9% | Good | Comparative Example |

From the results indicated in Fig. 1 to Fig. 3 and Table 2, it was confirmed that by using the cortisol-BSA conjugate of the present invention is used, cortisol can be analyzed by immunoassay with high sensitivity in a clinically significant cortisol concentration range (2 µg/dL to 30 µg/dL).

### (Example 2)

Cortisol-BSA conjugates 4, 5 and 6 having cortisol label numbers of 11.0, 15.7 and 16.9 were produced in the same manner as in Example 1, except that the ratio of the cortisol oxime derivative 1 and the amount of BSA was varied.

Samples containing cortisol at 5 levels of concentration (1.5 µg/dL, 4.3 µg/dL, 10.1 µg/dL, 20.5 µg/dL, and 33.2 µg/dL) were provided, and an immunoassay was carried out in the same manner as in Example 1. Thus, the fluorescence signal values at the various concentrations were obtained. Furthermore, calibration curves were obtained in the same manner as in Example 1. In the same manner as in Example 1, the reciprocal numbers of the gradient of the calibration curves in the low concentration range, and the deviations from the calibration curves in the high concentration range were evaluated. The reciprocal number of the gradient of the calibration curve at a cortisol concentration of 4.3 µg/dL in a low concentration range was determined, and for the cortisol concentration in the high concentration range, the deviation from the calibration curve at a cortisol concentration of 33.2 µg/dL was determined so as to guarantee measurement at 30 µg/dL, which is a clinically significant concentration. The results are shown in Table 3.

### [Table 3]

**Table 3: Gentleness of calibration curve in low concentration range, and deviation of average measured values from calibration curves in high concentration range**

| Cortisol/BSA ratio | Reciprocal of calibration curve gradient in low concentration range | | Deviation from calibration curve in high concentration range | | |
|---|---|---|---|---|---|
| | (Reference 2.0 or less | Result | (Reference 4% or less) | Result | |
| 16.9 | 1.4 | Good | -3.2% | Good | Example |
| 15.7 | 1.6 | Good | -3.5% | Good | Comparative Example |
| 11.0 | 1.2 | Good | -5.3% | NG | Comparative Example |

From the results of Table 3, it was confirmed that by using the cortisol-BSA conjugate having the cortisol/albumin ratio (cortisol/BSA ratio) of the present invention, cortisol can be analyzed by immunoassay with high sensitivity at a cortisol concentration in the range of 2 µg/dL to 30 µg/dL.

## Claims

1. A base plate for cortisol immunoassay, comprising a cortisol albumin conjugate having a cortisol/albumin ratio of from 12 to 20.

2. A base plate for cortisol immunoassay according to Claim 1, wherein the cortisol albumin conjugate has a cortisol/albumin ratio of from 14 to 18.

3. A base plate for cortisol immunoassay according to Claim 1 or Claim 2, wherein the albumin in the cortisol albumin conjugate is bovine serum albumin.

4. A base plate for cortisol immunoassay according to any preceding claim, wherein the cortisol albumin conjugate is immobilized to a metal film provided on the base plate.

5. A method for measuring cortisol in a test sample, the method comprising:
(1) bringing a base plate for cortisol immunoassay which has a cortisol albumin conjugate having a cortisol/albumin ratio of from 12 to 20 immobilized thereon, into contact with anti-cortisol antibody-labeled fluorescent particles and a test sample containing cortisol; and
(2) measuring the fluorescence based on the anti-cortisol antibody-labeled fluorescent particles bound to the base plate.

6. A method according to Claim 5, wherein the albumin is bovine serum albumin.

7. A method according to Claim 5 or Claim 6, wherein the measurement is carried out for a test sample having a cortisol concentration of 2 µg/dL to 30 µg/dL.

8. A method according to any one of Claims 5 to 7, wherein fluorescence is measured in step (2) by surface plasmon fluorescence measurement or epi-illumination fluorescence measurement.

## Patentansprüche

1. Basisplatte für einen Cortisol-Immunoassay, umfassend ein Cortisol-Albumin-Konjugat mit einem Cortisol/Albumin-Verhältnis von 12 bis 20.

2. Basisplatte für einen Cortisol-Immunoassay gemäß Anspruch 1, worin das Cortisol-Albumin-Konjugat ein Cortisol/Albumin-Verhältnis von 14 bis 18 aufweist.

3. Basisplatte für einen Cortisol-Immunoassay gemäß Anspruch 1 oder Anspruch 2, worin das Albumin in dem Cortisol-Albumin-Konjugat bovines Serum Albumin ist.

4. Basisplatte für einen Cortisol-Immunoassay gemäß irgendeinem vorstehenden Anspruch, worin das Cortisol-Albumin-Konjugat auf einem Metallfilm immobilisiert ist, der auf der Basisplatte vorgesehen ist.

5. Verfahren zur Messung von Cortisol in einer Testprobe, wobei das Verfahren umfasst:
(1) in Kontakt bringen einer Basisplatte für einen Cortisol-Immunoassay, die ein Cortisol-Albumin-Konjugat mit einem Cortisol/Albumin-Verhältnis von 12 bis 20 hierauf immobilisiert aufweist, mit einem mit Anti-Cortisol-Antikörper-gelabelten Fluoreszenzpartikeln und einer Testprobe, die Cortisol enthält; und
(2) Messen der Fluoreszenz, bezogen auf die Anti-Cortisol-Antikörper-gelabelten Fluoreszenzpartikel, die an die Basisplatte gebunden sind.

6. Verfahren gemäß Anspruch 5, worin das Albumin bovines Serum Albumin ist.

7. Verfahren gemäß Anspruch 5 oder Anspruch 6, worin die Messung für eine Testprobe mit einer Cortisol-Konzentration von 2 µg/dl bis 30 µg/dl durchgeführt wird.

8. Verfahren gemäß irgendeinem der Ansprüche 5 bis 7, worin im Schritt (2) die Fluoreszenz durch eine Oberflächenplasmon-Fluoreszenzmessung oder durch eine Epi-Illuminations-Fluoreszenzmessung gemessen wird.

## Revendications

1. Plaque de base pour dosage immunologique de cortisol comprenant un conjugué de cortisol et d'albumine ayant un rapport cortisol/albumine de 12 à 20.

2. Plaque de base pour dosage immunologique de cortisol selon la revendication 1, dans laquelle le conjugué de cortisol et d'albumine a un rapport cortisol/albumine de 14 à 18.

3. Plaque de base pour dosage immunologique de cortisol selon la revendication 1 ou la revendication 2, dans laquelle l'albumine du conjugué de cortisol et d'albumine est de l'albumine de sérum bovin.

4. Plaque de base pour dosage immunologique de cortisol selon l'une quelconque des revendications précédentes, dans laquelle le conjugué de cortisol et d'albumine est immobilisé sur un film métallique appliqué sur la plaque de base.

5. Procédé de mesure du cortisol dans un échantillon d'essai, le procédé comprenant les étapes consistant à :
(1) amener une plaque de base pour dosage immunologique de cortisol qui a un conjugué de cortisol et d'albumine ayant un rapport cortisol/albumine de 12 à 20 qui y est immobilisé, en contact avec des particules fluorescentes marquées par un anticorps anti-cortisol et un échantillon d'essai contenant du cortisol ; et
(2) mesurer la fluorescence sur la base des particules fluorescentes marquées par l'anticorps anti-cortisol liées à la plaque de base.

6. Procédé selon la revendication 5, dans lequel l'albumine est de l'albumine de sérum bovin.

7. Procédé selon la revendication 5 ou la revendication 6, dans lequel la mesure est effectuée pour un échantillon d'essai ayant une concentration de cortisol de 2 µg/dL à 30 µg/dL.

8. Procédé selon l'une quelconque des revendications 5 à 7, dans lequel la fluorescence est mesurée à l'étape (2) par mesure de la fluorescence des plasmons de surface ou par mesure de la fluorescence d'épi-illumination.
